# EUROPEAN PATENT SPECIFICATION

(11) **EP 3 445 249 B1**
(45) Date of publication and mention of the grant of the patent: **15.04.2020**
(21) Application number: 17720030.0
(22) Date of filing: 18.04.2017
(51) Int. Cl.: A61B 8/00, G06T 19/00, G02B 27/01, A61B 6/00

(54) **ULTRASOUND IMAGING PROBE POSITIONING**
ULTRASCHALLBILDGEBUNGSSONDENPOSITIONIERUNG
POSITIONNEMENT DE SONDE D'IMAGERIE À ULTRASONS

(30) Priority: 19.04.2016 US 201662324697 P; 19.10.2016 EP 16194671
(43) Date of publication of application: 27.02.2019
(73) Proprietor: Koninklijke Philips N.V., 5656 AG Eindhoven (NL)
(72) Inventor: DJAJADININGRAT, Johan Partomo, 5656 AE Eindhoven (NL); DU, Jia, 5656 AE Eindhoven (NL); CHAN, Raymond, 5656 AE Eindhoven (NL); CHEN, Njin-Zu, 5656 AE Eindhoven (NL)
(74) Representative: Mumbrú Forn, José
(86) International application number: PCT/EP2017/059086
(87) International publication number: WO 2017/182417

(56) References cited:
- WO-A1-2016/032298
- US-A1- 2004 019 270
- US-A1- 2006 253 031
- US-A1- 2013 237 811
- US-A1- 2014 236 012
- US-A1- 2015 305 718

## Description

### FIELD OF THE INVENTION

The present invention relates to an ultrasound imaging guidance system for guiding an operator of an ultrasound imaging system.

The present invention further relates to an ultrasound imaging system including such an ultrasound imaging guidance system.

The present invention further relates to an ultrasound imaging support system for providing support information to such an ultrasound imaging guidance system.

The present invention further relates to a method of guiding the operation of an ultrasound imaging system comprising an ultrasound probe.

The present invention further relates to a computer program product for implementing the method of guiding the operation of an ultrasound imaging system comprising an ultrasound probe on the ultrasound imaging guidance system.

The present invention further relates to a method of generating guidance information for operating an ultrasound imaging system comprising an ultrasound probe.

The present invention further relates to a computer program product for implementing the method of generating guidance information for operating an ultrasound imaging system comprising an ultrasound probe on the ultrasound imaging support system.

### BACKGROUND OF THE INVENTION

Ultrasound imaging forms an integral part of the diagnostic tools used by medical practitioners all across the world. Nowadays, ultrasound imaging systems are routinely used by many medical practitioners including medical practitioners in remote locations, e.g. rural areas of the developing world, as well as by ambulatory medical support staff. One of the challenges for such medical practitioners is to correctly use the ultrasound imaging system to obtain useful diagnostic information from the ultrasound images that are captured. Some medical practitioners may not be as skilled in using such ultrasound imaging systems as others, which may compromise the image quality of the ultrasound images captured with such a system and/or may lead to the region of interest to be imaged being missed, which consequently leads to an incorrect or missed diagnosis of a medical condition.

US 2003/0083563 A1 discloses a system and method for streaming unprocessed medical image data from a medical imaging system to a remote terminal. A medical imaging system acquires medical image data, generates unprocessed medical image data, and then transmits the unprocessed medical image data to a remote terminal. The remote terminal receives the unprocessed medical image data, processes the data to render a medical image and displays the medical image to an operator at the remote terminal.

This prior art system and method can offer support for local medical practitioner by expert guidance of a more expert medical practitioner at the remote terminal. However, a remaining problem with this solution is that the local medical practitioner may be unable to generate medical image data of sufficient quality, for instance by inappropriate positioning of an ultrasound probe of an ultrasound imaging system. This may make it difficult for the remote expert to provide appropriate guidance to the local medical practitioner.

Document US 2013/237811 A1 may be considered to disclose a system comprising a remote ultrasound imaging support system and an ultrasound image guidance system; the ultrasound imaging guidance system for guiding an operator of an ultrasound imaging system comprising an ultrasound probe, the ultrasound imaging guidance system comprising: a transceiver adapted to receive ultrasound images generated by the operator with the ultrasound probe from the ultrasound imaging system; generate pose information of the ultrasound probe when capturing said ultrasound image; transmit a data stream to a remote ultrasound imaging support system; and receive target ultrasound probe pose information from the remote ultrasound imaging support system, said target ultrasound probe pose information being generated by the remote ultrasound imaging support system; the ultrasound image guidance system system further comprising a processor communicatively coupled to the transceiver and programmed to generate a virtual image of the ultrasound probe in a pose corresponding to the target ultrasound probe pose information, and a display device communicatively coupled to the processor and adapted to display the virtual image.

### SUMMARY OF THE INVENTION

The present invention seeks to provide an ultrasound imaging guidance system for supporting an ultrasound imaging system comprising an ultrasound probe that will assist the user of the ultrasound imaging system in correctly positioning the ultrasound probe.

The present invention further seeks to provide an ultrasound imaging system comprising such an ultrasound imaging guidance system.

The present invention further seeks to provide an ultrasound imaging support system that facilitates a remote expert to generate ultrasound probe positioning instructions for use of such an ultrasound imaging system.

The present invention further seeks to provide a method of supporting the operation of an ultrasound imaging system comprising an ultrasound probe that assists the user of the ultrasound imaging system in correctly positioning the ultrasound probe, as well as a computer program product for implementing such a method on an ultrasound imaging guidance system.

The present invention further seeks to provide a method of generating guidance information for operating an ultrasound imaging system comprising an ultrasound probe that facilitates a remote expert to generate ultrasound probe positioning instructions for use of such an ultrasound imaging system as well as computer program product for implementing such a method of an ultrasound imaging support system.

According to an aspect, there is provided an ultrasound imaging guidance system for supporting an ultrasound imaging system comprising an ultrasound probe, the ultrasound imaging guidance system comprising a transceiver adapted to receive target ultrasound probe pose information generated by a remote ultrasound imaging support system, said target ultrasound probe pose information being derived from a data stream transmitted to the remote ultrasound imaging support system, said data stream including a sequence of ultrasound images generated with the ultrasound probe and an indication for each ultrasound image of the actual pose of the ultrasound probe when capturing said ultrasound image; a processor communicatively coupled to the transceiver and programmed to generate a virtual image of the ultrasound probe in a pose corresponding to the target ultrasound probe pose information; and a display device communicatively coupled to the processor and adapted to display the virtual image.

The present invention is based on the insight that a locally generated ultrasound image sequence may be complemented with ultrasound probe pose information. A remote expert may select a particular part of the sequence, for example an ultrasound image from the sequence. The pose of the ultrasound probe associated with that particular ultrasound image from the ultrasound probe pose may be communicated back to the ultrasound imaging guidance system as a target pose for the ultrasound probe, either directly or via the ultrasound imaging system, where this target pose is displayed as a virtual image of the ultrasound probe in the desired pose, such that the local practitioner can position the ultrasound probe in accordance with this virtual image to aid the local practitioner in generating an ultrasound image of a sufficient image quality to facilitate the local practitioner (or the remote expert) to make a sound diagnosis. In addition, such a guidance system can be used to provide remote training, e.g. to students practising on a patient substitute, e.g. a volunteer, corpse or the like.

In an embodiment, the ultrasound imaging guidance system takes the form of a head-mountable device including the display device such that the virtual image may be presented as augmented reality to the local practitioner, which has the advantage that the practitioner can position the virtual image on the body of the patient to be imaged and overlay the actual ultrasound probe position with the virtual image to obtain a particularly accurate positioning of the ultrasound probe. Alternatively, the ultrasound imaging guidance system may take the form of a tablet computer or a (distributed) computer system in which the display device is separated from the transducer and/or the processor.

The ultrasound imaging system is adapted to transmit the data stream to the remote ultrasound imaging support system. Alternatively, the transceiver may be further adapted to receive the sequence of ultrasound images from the ultrasound imaging system; generate the actual pose information of the ultrasound probe for each of the ultrasound images; and transmit said data stream to the remote ultrasound imaging support system. This has the advantage that the remote ultrasound imaging support system only has to communicate with a single system. In another embodiment, the ultrasound imaging system is adapted to relay the data stream generated by the ultrasound imaging guidance system to the ultrasound imaging support system and/or to relay the target ultrasound probe pose information generated by the remote ultrasound imaging support system to the transducer of the ultrasound imaging guidance system.

In an embodiment, the sequence of ultrasound images comprises a sequence of 2-D slices for constructing a 3-D ultrasound volume.

In at least some embodiments, the processor may be adapted to derive the indication of the actual pose of the ultrasound probe for each slice based on a patient body model. For example, the processor may be adapted to recalculate the pose of the ultrasound probe for a slice of a 3-D image volume from the probe pose during capturing of the 3-D image volume and the slice direction of the 3-D slice.

Alternatively, the ultrasound imaging guidance system may further comprise a probe pose detector adapted to generate the indication of the actual pose of the ultrasound probe when capturing an ultrasound image in said sequence. For example, the probe pose detector may comprise a camera adapted to capture an image of the actual pose of the ultrasound probe when generating an ultrasound image of said sequence. Alternatively, the the ultrasound probe may include one or more orientation sensors adapted to generate the ultrasound probe pose information, e.g. one or more accelerometers, gyroscopes, Hall sensors or the like.

In an embodiment, the transceiver is further adapted to receive one of the ultrasound images of said sequence from the remote ultrasound imaging support system, said ultrasound image including a highlighted region; and the display device is further adapted to display the ultrasound image including the highlighted region. By sharing highlighted images between the ultrasound imaging support system and the ultrasound imaging guidance system, the local practitioner may be supported by the remote expert in the evaluation of the ultrasound images captured with the ultrasound imaging system, thereby further aiding patient diagnosis.

According to another aspect, there is provided an ultrasound imaging system comprising an ultrasound probe and the ultrasound imaging guidance system of any of the herein described embodiments. Such an ultrasound imaging system benefits from the provision of ultrasound probe pose guidance by the ultrasound imaging guidance system, thereby providing an ultrasound imaging system that may be appropriately operated more easily.

According to yet another aspect, there is provided an ultrasound imaging support system comprising a transceiver adapted to receive a data stream including a sequence of ultrasound images generated with an ultrasound probe of an ultrasound imaging system and an indication for each ultrasound image of the actual pose of the ultrasound probe when capturing said ultrasound image; a processor communicatively coupled to the transceiver; a display device communicatively coupled to the processor; and a user interface communicatively coupled to the processor; wherein the processor is programmed to control the display device to display the sequence of ultrasound images; receive a user input from the user interface indicative of an image selection from said sequence of ultrasound images; and generate target ultrasound probe pose information from the received indications of the actual pose of the ultrasound probe and the received image selection, wherein the transceiver is further adapted to transmit the target ultrasound probe pose to a remote ultrasound imaging guidance system associated with the ultrasound imaging system.

Such an ultrasound imaging support system makes it possible for an ultrasound expert to receive a data stream of ultrasound images from a remote location, such that the expert can provide user input indicative of a preferred ultrasound image, e.g. an ultrasound image providing the best probe pose for imaging a region of interest of the patient being investigated, in the sequence, from which the ultrasound imaging support system can determine the required ultrasound probe pose in order to capture the preferred ultrasound image from the pose information for each of the ultrasound images of the pose of the ultrasound probe in which the ultrasound image was captured included in the data stream and transmit this ultrasound probe pose to the remote ultrasound imaging guidance system.

The user-specified image selection may comprise a selected ultrasound image from the sequence of ultrasound images or a 2-D image slice of a 3-D ultrasound volume defined by the sequence of ultrasound images. Such a 2-D image slice does not have to be present in the received data stream but instead may be generated by the expert by re-slicing the 3-D ultrasound volume in a direction different to the original slicing direction of the 2-D image slices in the data stream.

The processor of the ultrasound image support system may be further programmed to receive a further user input from the user interface indicative of a selected area within a selected ultrasound image from said sequence of ultrasound images; and generate a highlighted region in the selected ultrasound image corresponding to the selected area, wherein the transceiver may be further adapted to transmit the selected ultrasound image including the highlighted region to the remote ultrasound imaging guidance system. In this manner, the local practitioner operating the ultrasound imaging system may be further guided by the remote expert by highlighting areas of interest in a particular ultrasound image produced with the ultrasound imaging system to assist the local practitioner in focusing on the relevant parts of this ultrasound image.

According to another aspect, there is provided a method of supporting the operation of an ultrasound imaging system comprising an ultrasound probe; the method comprising receiving target ultrasound probe pose information derived from a data stream including a sequence of ultrasound images generated with the ultrasound probe and an indication for each ultrasound image of the actual pose of the ultrasound probe when capturing said ultrasound image from a remote ultrasound imaging support system; generating a virtual image of the ultrasound probe in a pose corresponding to the target ultrasound probe pose information; and displaying the virtual image. As explained above, this assists the local practitioner in correctly positioning the ultrasound probe on the patient's body, thereby increasing the likelihood of the ultrasound imaging system and the local practitioner correctly diagnosing the patient.

The method may further comprise receiving the sequence of ultrasound images from the ultrasound imaging system; generating the actual pose information of the ultrasound probe for each of the ultrasound images; and transmitting said data stream to a remote ultrasound imaging support system, which has the advantage that the remote ultrasound image support system can communicate with a single point of contact, i.e. a single system.

According to another aspect, there is provided a computer program product comprising a computer readable storage medium having computer readable program instructions embodied therewith for, when executed on the processor of the ultrasound imaging guidance system as described in this application, cause the processor to implement the steps of the method of supporting the operation of an ultrasound imaging system comprising an ultrasound probe as described in this application.

According to another aspect, there is provided a method of generating guidance information for operating an ultrasound imaging system comprising an ultrasound probe, the method comprising receiving a data stream including a sequence of ultrasound images generated with the ultrasound probe and an indication for each ultrasound image of the actual pose of the ultrasound probe when capturing said ultrasound image; displaying the sequence of ultrasound images; receiving a user input indicative of an image selection from said sequence of ultrasound images, wherein the image selection comprises a selected ultrasound image from the sequence of ultrasound images or an 2-D image slice of a 3-D ultrasound volume defined by the sequence of ultrasound images; generating target ultrasound probe pose information from the received indications of the actual pose of the ultrasound probe and the received user input; and transmitting the target ultrasound probe pose information to a remote ultrasound imaging guidance system associated with the ultrasound imaging system. As explained above, such a method facilitates an expert in a location remote to the ultrasound imaging system to provide guidance as to how the ultrasound imaging system should be correctly used, i.e. by providing a target pose of the ultrasound probe.

According to another aspect, there is provided a computer program product comprising a computer readable storage medium having computer readable program instructions embodied therewith for, when executed on the processor of the ultrasound imaging support system as described in this application, cause the processor to implement the steps of the method of generating guidance information for operating an ultrasound imaging system comprising an ultrasound probe as described in this application.

### BRIEF DESCRIPTION OF THE DRAWINGS

Embodiments of the invention are described in more detail and by way of non-limiting examples with reference to the accompanying drawings, wherein:
FIG. 1 schematically depicts a principle according to embodiments of the present invention;
FIG. 2 schematically depicts an aspect of a further embodiment of the present invention;
FIG. 3 schematically depicts an ultrasound imaging guidance system according to an embodiment;
FIG.4 schematically depicts an ultrasound imaging guidance system according to another embodiment;
FIG. 5 is a flowchart of an ultrasound imaging support method according to an embodiment;
FIG. 6 schematically depicts an ultrasound imaging support system according to an embodiment; and
FIG. 7 is a flowchart of an ultrasound imaging guidance method according to an embodiment.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

In the present application, where reference is made to pose information for an ultrasound probe, this is intended to cover information from which the orientation and location of the ultrasound probe can be derived. For example, such pose information may include position information, which may be defined in Cartesian coordinates (x, y, z coordinates) or an equivalent thereof, as well as angular information, which may be defined in Euler angles (Rₓ, R_{y}, R_{z}) or an equivalent thereof. Any suitable representation of such a pose may be deployed.

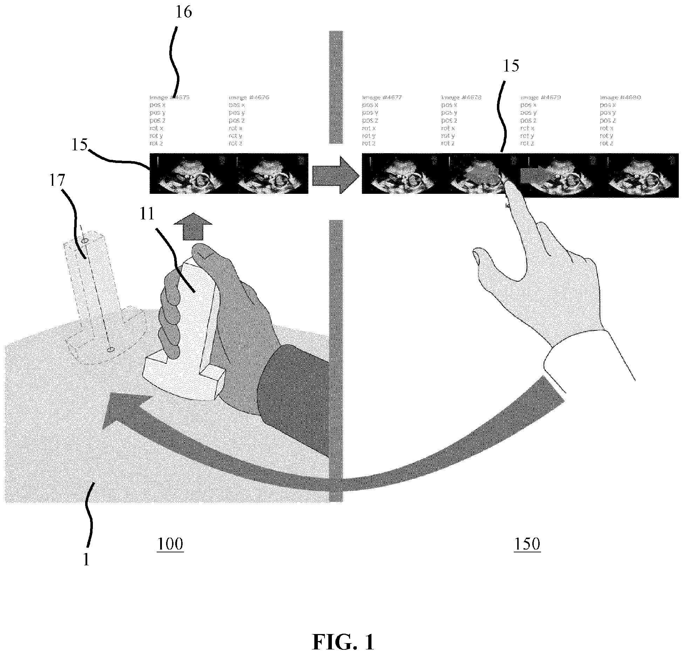

FIG. 1 schematically depicts a principle according to embodiments of the present invention. According to this principle, a medical practitioner in a first location 100, such as a rural location, an ambulatory location such as an ambulance or the like, and so on, may use an ultrasound probe 11 of an ultrasound imaging system on a body part of a patient 1 in order to generate a sequence of ultrasound images 15. The medical practitioner in the first location 100 may not be experienced in using such an ultrasound imaging system and may therefore be unsure of the correct operation, i.e. positioning, of the ultrasound probe 11 relative to the body part of the patient 1.

In accordance with embodiments of the present invention, the sequence of ultrasound images 15 generated by the medical practitioner in the first location 100 may be transmitted in a data stream to an expert in the use of such ultrasound imaging system is in a second location 150, which may be a location that is geographically remote to the first location 100 to such an extent that the expert in the second location 150 cannot easily support the medical practitioner in the first location 100 in person. For example, the first location 100 may be a rural location and the second location 150 may be a hospital or other medical facility in a city at a relatively large distance from the rural location.

Each ultrasound image 15 in the data stream is supplemented by the pose information for the ultrasound probe 11 when capturing the ultrasound image 15, e.g. a pose relative to the body part of the patient 1. The pose of the ultrasound probe 11 may be determined in any suitable manner as will be described in more detail below. The pose information of the ultrasound probe may be included in the data stream in any suitable manner, e.g. each ultrasound image 15 may be tagged with metadata 16 specifying the pose of the ultrasound probe 11 during capture of the image. For example, the pose information may define the position and rotation or tilt angle of the ultrasound probe 11, e.g. in a Cartesian coordinate system using Euler angles by way of non-limiting example.

The data stream including the sequence of ultrasound images 15 and associated ultrasound probe pose information 16 may be transmitted from the first location 100 to the second location 150 in any suitable manner, e.g. over the Internet or over a mobile communications link operating a mobile communications standard such as GMS or UMTS over a 2G, 3G, 4G or higher generation mobile communications network, etcetera.

The data stream including the sequence of ultrasound images 15 and associated ultrasound probe pose information 16 may be received by the expert in the second location 150 and displayed on the display device of an ultrasound imaging support system, which will be explained in more detail below. For example, the expert may operate the display device to scroll through the sequence of ultrasound images 15, e.g. using a user interface device such as a mouse or scroll ball, using a user interface device integral to the display device, e.g. a touch-sensitive screen, using a user interface in the form of speech recognition software, and so on, in order to select the ultrasound image 15 in the sequence that provides the best view of the part of the anatomy of the patient 1 under investigation, e.g. a clear view of an artery or vein, part of an organ such as the stomach, kidney, liver, bowel or heart, and so on.

The ultrasound imaging support system identifies the ultrasound image 15 selected by the expert in the second location 150 in the data stream received from the first location 100 and retrieves the pose information 16 of the ultrasound probe 11 that belongs to the selected ultrasound image 15, i.e. that specifies the pose of the ultrasound probe 11 in which the selected ultrasound image 15 was captured and transmits this pose information 16 to an ultrasound imaging guidance system in the first location 100, which will be described in more detail below. Alternatively, the ultrasound imaging support system may transmit the target ultrasound probe pose information in the form of an identifier of the expert-selected ultrasound image 15 to the ultrasound imaging guidance system, such that the ultrasound imaging guidance system may locally retrieve the appropriate pose information 16 of the ultrasound probe 11 by extracting this pose information from the metadata associated with the ultrasound image 15 identified by the identifier transmitted by the ultrasound imaging support system in the second location 150.

The ultrasound imaging guidance system in the first location 100 receives the pose information 16 associated with the expert-selected ultrasound image 15 in the form of the actual pose data of the ultrasound probe 11 or in the form of an identifier of the expert-selected ultrasound image 15 from which the ultrasound imaging guidance system may retrieve the actual pose data of the ultrasound probe 11 as explained above and constructs a virtual image 17 of the ultrasound probe 11 representing the actual pose of the ultrasound probe 11 during the time of capture of the expert-selected ultrasound image 15.

The ultrasound imaging guidance system typically comprises a display device onto which the virtual image 17 is displayed. As will be explained in more detail below, in preferred embodiments the display device may form part of an augmented reality device, e.g. a head-mountable computing device, such that the medical practitioner in the remote location 100 can create an overlay including the virtual image 17 over a scene viewed by the medical practitioner, which has the advantage that the virtual image 17 may be positioned in the appropriate position on the body of the patient 1, such that the medical practitioner simply may reposition the ultrasound probe 11 by positioning it coinciding with the virtual image 17. In preferred embodiments, the virtual image 17 is a 3-D image, e.g. a holographic representation of the ultrasound probe 11 although other suitable representations may also be contemplated. Alternatively, the virtual image 17 may be displayed on a display device such as a tablet computer or a monitor, which may be mounted on an arm, tripod or the like such that the medical practitioner may observe the virtual image 17 displayed on the display device whilst simultaneously observing the actual pose of the ultrasound probe 11 on the body of the patient 1.

In an embodiment, the indication of the pose information 16 submitted by the ultrasound imaging support system corresponding to the ultrasound image 15 selected by the expert in second location 150 may be supplemented with an ultrasound image 15, e.g. the expert-selected ultrasound image 15 in which a region of interest is highlighted by the expert. For example, the expert may highlight the region of interest in the ultrasound image 15 to draw attention of the medical practitioner in first location 100 to the region in the ultrasound image 15 that should be brought into focus with the ultrasound probe 11, e.g. the region in the ultrasound image 15 of diagnostic relevance.

The medical practitioner and the expert may further share an ultrasound image 15, e.g. the ultrasound image 15 including the highlighted region, in which the expert and/or the medical practitioner may highlight a region in the ultrasound image 15 in real time, e.g. using a cursor or the like. This for example may be particularly advantageous in case of a further communications link between the medical practitioner in first location 100 and the expert in second location 150, e.g. a voice link by phone or over the Internet, as this facilitates effective discussion of the ultrasound image 15 under consideration by pointing to relevant areas in the ultrasound image 15 with the cursor.

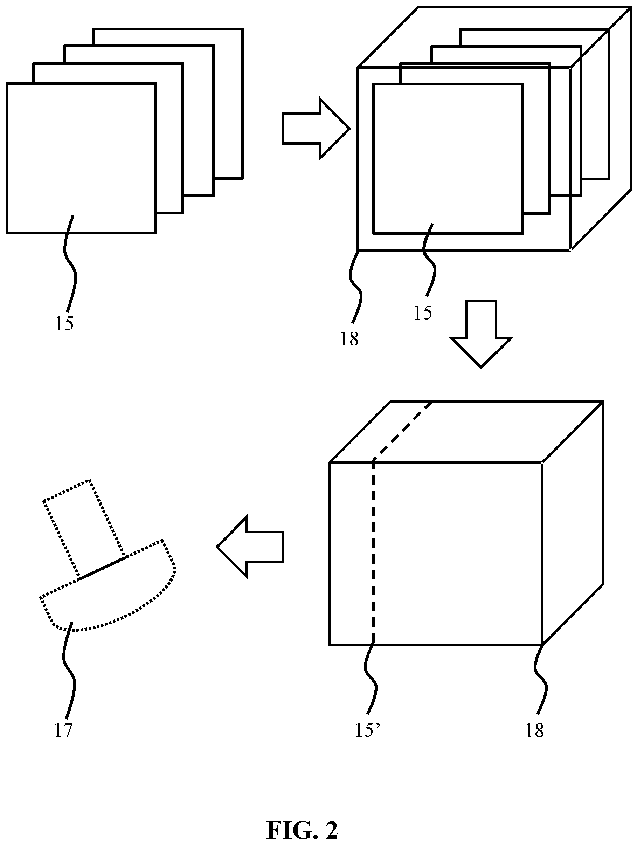

In an embodiment, the medical practitioner in the first location 100 may operate an ultrasound imaging system adapted to generate a 3-D volumetric ultrasound image with the ultrasound imaging system. This is typically is achieved by the medical practitioner moving the ultrasound probe 11 in a particular direction over a region of the body of the patient 1, during which the ultrasound probe 11 periodically captures a 2-D ultrasound image slice of the 3-D volumetric ultrasound image. As schematically depicted in FIG. 2, in this embodiment, the data stream transmitted from the first location 100 to the second location 150 comprises a plurality of such 2-D ultrasound image slices 15, from which the 3-D volumetric ultrasound image 18 may be constructed, e.g. on the ultrasound imaging support system in the second location 150. The expert may select one of the 2-D ultrasound image slices 15 for regeneration by the medical practitioner in the first location 100 as previously explained.

Alternatively, as is well-known per se, such a 3-D volumetric ultrasound image 18 may be re-sliced following its construction, e.g. to define a volume slice 15', which may be sliced in a different direction compared to the original 2-D ultrasound image slices 15. The expert in the second location 150 may for instance perform such a re-slicing of the 3-D volumetric ultrasound image 18 in order to obtain a slice of this 3-D volumetric ultrasound image that contains the desired body feature of the patient 1 under investigation.

Because such a reconstructed volume slice 15' typically has a lower resolution (e.g. as a consequence of image processing required to create the reconstructed volume slice 15') than the original ultrasound image slices 15, the expert may request that the medical practitioner (sonographer) in the first location 100 repositions the ultrasound probe 11 corresponding to the volume slice 15'such that a high resolution 2-D image corresponding to the reconstructed volume slice 15' may be captured with the ultrasound system including the ultrasound probe 11.

To this end, the ultrasound imaging support system may extrapolate the target pose of the ultrasound probe 11 for generating this high resolution 2-D image from the pose information 16 associated with the respective original 2-D ultrasound image slices 15 as received in the data stream from the first location 100. For example, the ultrasound imaging support system may extrapolate the pose of the ultrasound probe 11 and the direction in which the ultrasound probe 11 was moved in order to capture the sequence of 2-D ultrasound image slices 15 from the received pose information 16 and may transform this orientation and direction by constructing a transformation matrix based on the difference between the original direction in which the ultrasound probe was moved leading to the stacking direction of the 2-D ultrasound image slices in the 3-D volumetric ultrasound image 18 and the slicing direction of the volume slice 15'.

The ultrasound imaging support system in the second location 150 may send the original ultrasound probe pose (or an indication thereof in the form of an identifier of a particular 2-D ultrasound image slice 15 as previously explained) together with this transformation matrix to the ultrasound imaging guidance system in the first location 100 such that the ultrasound imaging guidance system can generate the virtual image 17 of the desired pose of the ultrasound probe 11 as previously explained or alternatively the ultrasound imaging support system may perform this transformation and simply send the transformed pose of the ultrasound probe 11 to the ultrasound imaging guidance system in the first location 100 for construction of the virtual image 17.

The ultrasound image generated with the ultrasound probe 11 in the pose as specified by the virtual image 17 may be shared between the medical practitioner in the first location 100 and the expert in the second location 150 as previously explained such that areas of interest in this ultrasound image, e.g. highlighted areas using a cursor or the like may be discussed or otherwise identified between the medical practitioner and the expert. Alternatively or additionally, the reconstructed volume slice 15' may be displayed on the ultrasound imaging guidance system to assist the medical practitioner in the first location 100 in reproducing the reconstructed volume slice 15' with the ultrasound system including the ultrasound probe 11.

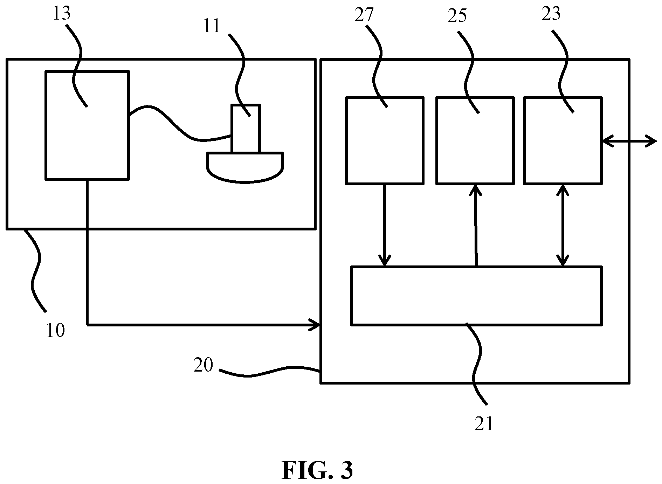

FIG. 3 schematically depicts an embodiment of an ultrasound imaging guidance system 20 to support an ultrasound imaging system 10 including the ultrasound probe 11 connected to a console 13 in the first location 100. The ultrasound imaging guidance system 20 typically comprises a processor 21 that is communicatively coupled to a transceiver 23 and a display device 25. Optionally, the ultrasound imaging system 10 may further comprise an ultrasound probe pose detector 27 communicatively coupled to the processor 21 to detect the pose of the ultrasound probe 11 during capture of an ultrasound image 15 as explained above.

The processor 21 may be any suitable processor, e.g. a general purpose processor or an application specific integrated circuit (ASIC). The processor may be programmed, e.g. using a computer program product including appropriate computer program code instructions, to generate the virtual image 17 of the ultrasound probe 11 in a pose corresponding to the target ultrasound probe pose information received from the ultrasound imaging support system via the transducer 23. The processor 21 in some embodiments may be a processor arrangement comprising multiple processors, e.g. a graphics processor to control the display device 25 and a signal processor to generate the virtual image 17 to be rendered by the graphics processor.

In the context of the present application, a transducer may be any device or component capable of communicating data over a data communications link such as a data communications network. The transducer may be adapted to establish a wired or wireless data communications link; for example, the transducer may be adapted to communicate the data using a short-range wireless communication protocol such as Wi-Fi, Bluetooth or a NFC protocol, a long-range wireless communication protocol such as GSM or UMTS, a wired communication protocol such as Ethernet, and so on. Any existing data communication protocol may be deployed by the transducer.

In the context of the present application, the display device 25 may be a component integral to a computing device such as a tablet computer or laptop computer or may be a stand-alone device that is connected via cable or the like to a separate component housing the processor 21. In a particularly preferred embodiment, which will be described in more detail below, the display device 25 forms part of a head-mountable device implementing the ultrasound imaging guidance system 20.

The probe pose detector 27 in some embodiments may be implemented as a camera (or a plurality of cameras) arranged to capture an image (or plurality of images) of the ultrasound probe 11 during capture of an ultrasound image 15. The image (or plurality of images) may be forwarded to the processor 21, which may be adapted to derive the probe pose from the captured image or images. An example of such a technique is disclosed in US 2003/0055335 A1. For example, the processor 21 may use a patient body model for the patient 1 to define a reference frame for the ultrasound probe 11 and determine the pose of the probe relative to this patient body model. In embodiments, the processor 21 may implement the patient body model as a static model although in alternative embodiments of the processor 21 may implement the patient body model as a dynamic model in which the model is updated in accordance with body movements of the patient 1 captured with the camera (or plurality of cameras). The provisioning of such a patient body model is well known per se. For example, a static patient body model may be captured using a 3D depth camera optionally supplemented with one or more stereotactic markers or utilizing bodily landmarks on the patient's body. Such a patient body model may be updated in accordance with monitored patient body movement, e.g. using a camera such as a Kinetic camera to keep the patient body model up to date.

The ultrasound imaging guidance system may include or have access to a data storage device (not shown) such as a memory, a hard disk, optical disk, cloud storage, network-attached storage, storage area network, and so on, which data storage device for example may store data of relevance to the processor 21, e.g. data pertaining to the patient body model.

The ultrasound probe 11 may comprise a visible marker that may be captured by the one or more cameras and recognised in the image or images generated by the one or more cameras by the processor 21. The processor 21 may use the recognised visible marker as an alignment aid for determining the pose of the ultrasound probe 11 relative to the body of the patient 1, e.g. relative to the patient body model. Alternatively, the processor 21 may utilise a CAD model of the ultrasound probe 11, which may be stored in the previously mentioned the data storage device, as a reference from which the pose of the ultrasound probe 11 may be calculated relative to the body of the patient 1. Alternatively, the pose of the ultrasound probe 11 relative to the body of the patient 1 may be determined using tracking techniques based on infrared, magnetic, ultrasound or radar tracking, for example. Any suitable tracking technique may be contemplated.

It should be understood that the pose of the ultrasound probe 11 may be determined in any suitable manner. For example, the ultrasound probe 11 may contain one or more orientation sensors, e.g. one or more accelerometers, gyroscopes, Hall sensors or the like that may provide pose information to be processed on the ultrasound imaging system 10 or by the processor 21. Alternatively, the pose of the ultrasound probe 11 may be determined relative to the console 13 using electromagnetic tracking techniques as for instance utilized by Ascension Technologies.

Each of the ultrasound images 15 generated with the ultrasound imaging system 10 may be labelled with the probe pose of the ultrasound probe 11 during capture of that image. This may be achieved in any suitable manner. For example, the ultrasound imaging guidance system 20 may comprise a transducer, e.g. the transducer 23 or a further transducer, for establishing a communication link with the ultrasound imaging system 10, which may be a wired or wireless communication link. The ultrasound imaging guidance system 20 may communicate the determined probe pose information to the ultrasound imaging system 10 for labelling with the captured ultrasound image 15 by the ultrasound imaging system 10 or the ultrasound imaging system 10 may communicate the captured ultrasound image 15 to the ultrasound imaging guidance system 20 for labelling with the probe pose information by the processor 21. In further embodiments where the probe pose information is determined by the ultrasound imaging system 10, no communication between the ultrasound imaging 10 and the ultrasound imaging guidance system 20 may be necessary or alternatively, the ultrasound imaging system 10 may communicate the sequence of ultrasound images 15 including the probe pose metadata to the ultrasound imaging guidance system 20. Other suitable arrangements will be immediately apparent to the skilled person.

At this point, it is noted that the ultrasound imaging system 10 is not particularly limited and may be any suitable ultrasound imaging system, e.g. an ultrasound imaging system 10 operable to generate 2-D ultrasound images, 3-D ultrasound images, 4-D ultrasound images (3-D scans in a movie) and so on. As such ultrasound imaging systems are well-known per se, this is not explained in further detail for the sake of brevity only.

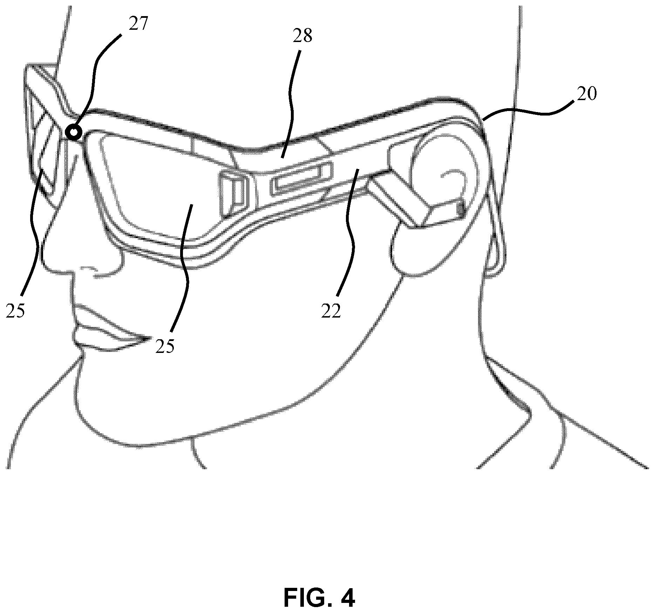

FIG. 4 schematically depicts a particularly preferred embodiment of the ultrasound imaging guidance system 20, in which this system is implemented in the form of a head-mountable computing device such that the virtual image 17 may be generated in the view of the medical practitioner in the first location to augment the reality (i.e. the actual view) of the medical practitioner, e.g. by superimposing the virtual image 17 onto this actual view.

In the context of the present application, a head-mountable computing device is a device that can be worn of the head of its user and provides the user with computing functionality. The head-mountable computing device may be configured to perform specific computing tasks as specified in a software application (app) that may be retrieved from the Internet or another computer-readable medium. Non-limiting examples of such head-mountable computing devices include smart headgear, e.g. eyeglasses, goggles, a helmet, a hat, a visor, a headband, or any other device that can be supported on or from the wearer's head, and so on.

The head-mountable computing device may include the processor 21 and transducer 23, e.g. in a component housing 22. The head mountable computing device may further include an image sensor or camera as the orientation detector 27 for capturing an image in a field of view of a wearer of the wearable computing device. The image sensor may be arranged such that when the head-mountable computing device is worn as intended, the image sensor aligns with the eyes of its wearer, i.e. produces a forward-facing sensor signal corresponding to the field of view of its wearer.

Such an image sensor or camera may be integral to the head-mountable computing device, such as integrated in a lens of a head-mountable computing device through which its wearer observes its field of view, in a lens holder or frame for such a lens, or in any other suitable structure of the head-mountable computing device in which the optical sensor aligns with the field of view of the wearer of the head-mountable computing device.

Alternatively, such an image sensor may be part of a modular wearable computing device, e.g. a head-mounted image sensor module communicatively coupled via a wired or wireless connection to one or more other modules of the head-mountable computing device, wherein at least some of the other modules may be worn on parts of the body other than the head, or wherein some of the other modules may not be wearable, but portable instead for instance.

The head-mountable computing device typically comprises at least one display module 25, which may be a see-through or transparent display module 25, under control of a discrete display controller (not shown). Alternatively, the display controller may be implemented by a processor 21 of the head-mountable computing device, as shown in FIG. 3.

The at least one display module 25 is typically arranged such that a wearer of the head-mountable computing device, e.g. the medical practitioner in the first location 100, can observe the virtual image 17 of the ultrasound probe 11 displayed on the at least one display module 25. Preferably, the at least one display module 25 is a see-through or transparent display module such that the wearer can observe at least a part of a field of view through the display module 25, e.g. the actual pose of the ultrasound probe 11. In an embodiment, the head-mountable computing device comprises a pair of display modules 25 including a first display module that can be observed by the right eye of the wearer and a second display module that can be observed by the left eye of the wearer. Alternatively, at least one display module 25 may be an opaque display module onto which an augmented reality scene of the field of view of its wearer is displayed, e.g. the field of vie augmented with the virtual image 17. To this end, the head-mountable computing device may include a camera for capturing the field of view of its wearer, as is well-known per se.

The first and second display modules may be controlled to display different images, e.g. to generate a stereoscopic image as is well-known per se in the art. Alternatively, an image may be generated on one of the first and second display modules only such that the wearer can observe the generated image with one eye and the actual field of view with the other eye. Both the first and second display modules may be see-through or transparent display modules. Alternatively, one of the first and second display modules may be a see-through or transparent display module, whereas the other display module is an opaque display module, i.e. a display module that is not transparent such that the wearer cannot see-through this display module.

The at least one display module 25 may be provided in any suitable form, such as a transparent lens portion. Alternatively, as shown in FIG. 4, the head-mountable computing device may comprise a pair of such a lens portions, i.e. one for each eye as explained above. The one or more transparent lens portions may be dimensioned such that substantially the entire field of view of the wearer is obtained through the one or more transparent lens portions. For instance, the at least one display module 25 may be shaped as a lens to be mounted in the frame 28 of the head-mountable computing device. Any other configuration known to the person skilled in the art may be contemplated.

It will be understood that the frame 28 may have any suitable shape and may be made of any suitable material, e.g. a metal, metal alloy, plastics material or combination thereof. Several components of the head-mountable computing device may be mounted in the frame 28, such as in a component housing 22 forming part of the frame 28. The component housing 22 may have any suitable shape, preferably an ergonomic shape that allows the head-mountable device to be worn by its wearer in a comfortable manner.

At this point, it is noted that the ultrasound imaging guidance system 20 may be a stand-alone system or may form a part of the ultrasound imaging system 10, e.g. may be integral to the ultrasound imaging system 10.

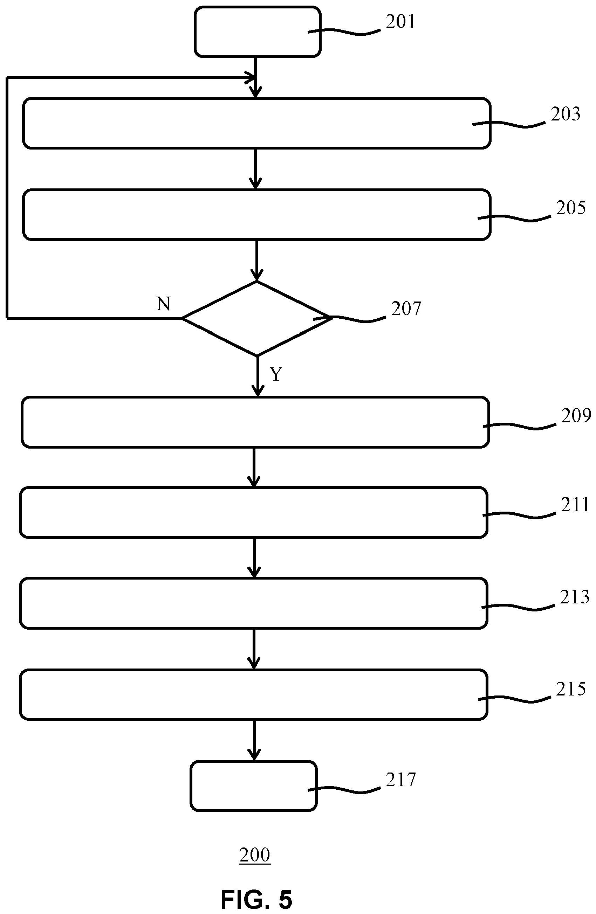

FIG. 5 schematically depicts a method 200 for guiding the operation of an ultrasound imaging system 10 comprising an ultrasound probe 11. The method 200 starts in 201 with the initialisation of the ultrasound imaging system 10 and ultrasound imaging guidance system 20 after which an ultrasound image of a patient 1 is captured in 203 with the ultrasound probe 11 of the ultrasound imaging system 10. At the same time, the pose of the ultrasound probe 11 whilst capturing the ultrasound image 15 in 203 is determined in 205 as previously explained. Steps 203 and 205 are repeated until all ultrasound images 15 of the sequence to be submitted to the ultrasound imaging support system in the second location 150 have been captured, as checked in 207. As previously explained, in some embodiments, the ultrasound images 15 may form 2-D slices of a 3-D volumetric ultrasound image.

Next, the data stream including the sequence of ultrasound images 15 generated with the ultrasound probe 11 and the indications for each ultrasound image of the actual pose of the ultrasound probe 11 when capturing said ultrasound image 15 is generated in 209, for example by the ultrasound imaging system 10 or the ultrasound imaging guidance system 20 and subsequently transmitted to the second location 150, e.g. to the ultrasound imaging support system in the second location 150 such that an ultrasound expert in the second location 150 can analyze the sequence of ultrasound images 15 and generate imaging guidance from which the ultrasound imaging guidance system 20 can generate the virtual image 17 as explained in more detail above.

In 213, the ultrasound imaging guidance system 20 receives the target probe pose information from the ultrasound imaging support system in the second location 150, e.g. directly or indirectly via an entity in the first location 100 in communication with the ultrasound imaging support system in the second location 150, e.g. via the ultrasound imaging system 10, after which the ultrasound imaging guidance system 20, i.e. the processor 21, generates the virtual image 17 of the target probe pose as derived from the information received in 213 and triggers the display of the generated virtual image 17 on the display device 215, after which the method 200 terminates in 217. It is noted for the avoidance of doubt that although the method 200 has been depicted as a series of sequential steps, it will be immediately apparent by the skilled person that at least some of the steps may alternatively be performed concurrently, i.e. in parallel.

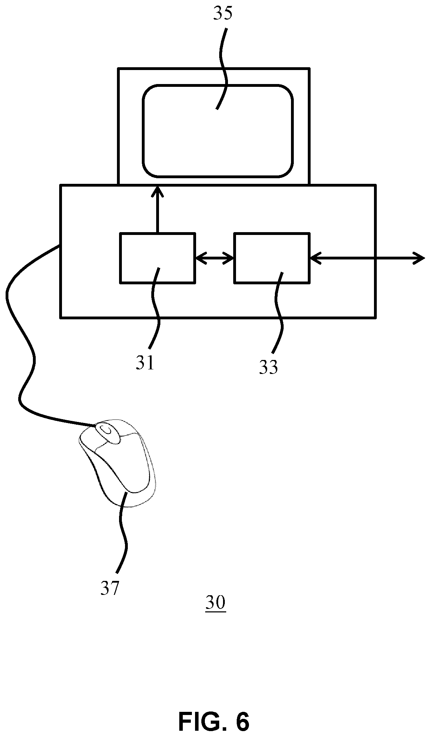

FIG. 6 schematically depicts an example embodiment of an ultrasound imaging support system 30 that receives the data stream including the ultrasound images 15 and probe pose information 16 for each ultrasound image 15 in the second location 150. The ultrasound imaging support system 30 typically comprises one or more processors 31 communicatively coupled to a transducer 33 arranged to receive the data stream. The one or more processors 31 may include a data processor programmed to process the data in the data stream, for example to generate a scrollable sequence of ultrasound images 15 and to control a display device 35 onto which this scrollable sequence of ultrasound images 15 may be displayed. Alternatively, the one or more processors 31 may include a separate processor in communication with the data processor adapted to control the display device 35, e.g. a graphics processor. The display device 35 may be any suitable display device, e.g. a display module integral to an apparatus further comprising the one or more processors 31 and the transducer 33, e.g. a tablet computer, laptop computer, a purpose-built console for processing ultrasound images 15, and so on, or alternatively may be a separate device that is coupled to a computing device or console via cable or the like.

The ultrasound imaging support system 30 further comprises one or more user interfaces 37, here symbolically depicted by a computer mouse by way of non-limiting example. The one or more user interfaces 37 may for example include one or more of a computer mouse, a keyboard, a touch screen, a trackball, a microphone for providing speech recognition software running on a processor 31 with spoken instructions, a camera for providing images of captured gestures or the like to gesture recognition software running on a processor 31, and so on. It should be understood that any existing user interface device may be used in conjunction with the ultrasound imaging support system 30.

In an embodiment, the ultrasound imaging support system 30 may at least partially be implemented as a head-mountable computing device such as the head-mountable computing device described in more detail above with the aid of FIG. 4.

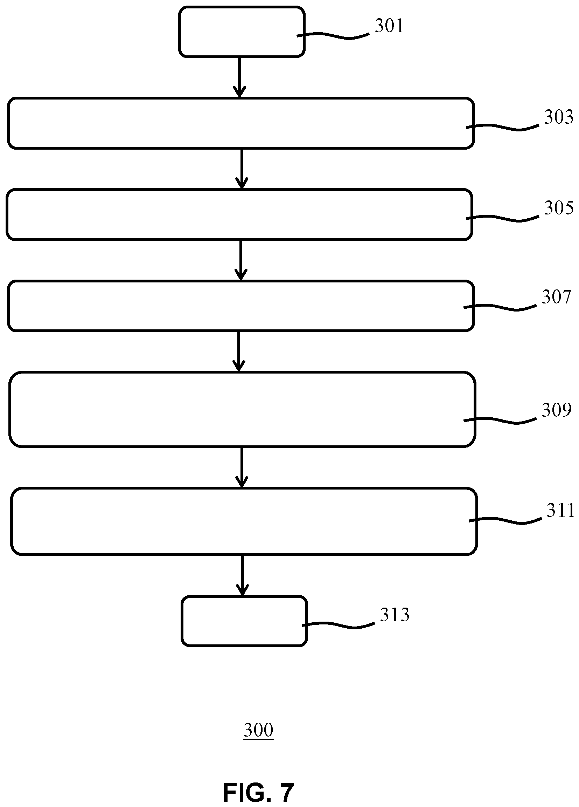

The ultrasound imaging support system 30 is typically programmed to implement a method 300 of generating guidance information for operating the ultrasound imaging system 10, an example embodiment of which is depicted by the flow chart in FIG. 7. The method 300 starts in 301 with the initialization of the ultrasound image support system 30 after which the data stream including the sequence of ultrasound images 15 generated with the ultrasound probe 11 and an indication 16 for each ultrasound image of the actual pose of the ultrasound probe when capturing said ultrasound image is received from the first location 100. Next, a processor 31 processes the received ultrasound images 15 and controls the display device 35 to display the sequence of ultrasound images 15 on the display device 35, e.g. as a scrollable sequence of ultrasound images 15 or as a volumetric (3-D) ultrasound image constructed from 2-D ultrasound image slices 15 received in the data stream.

In 307, the ultrasound imaging support system 30, i.e. a processor 31, receives a user input provided through one or more of the user interfaces 37, which user input is indicative of an image selection from said sequence of ultrasound images. For example, an expert in the second location 150 may select a particular ultrasound image 15 from the sequence of ultrasound images 15 because it provides the best view of a particular anatomical feature of interest or alternatively the expert may generate a 2-D image slice of a 3-D ultrasound volume defined by the sequence of ultrasound images 15. It is reiterated that such a 2-D image slice does not need to correspond to a 2-D image slice 15 in the data stream; instead, the expert may re-slice the 3-D image volume in a different direction to obtain a 2-D image slice 15' providing the desired view of a particular anatomical feature of interest.

In 309, the processor 31 of the ultrasound imaging support system 30 generates target ultrasound probe pose information from the received indications of the actual pose of the ultrasound probe and the received user input and transmits the target ultrasound probe pose information to the ultrasound imaging guidance system 20 associated with the ultrasound imaging system 10 in the first location 100, either directly or indirectly as previously explained.

The target ultrasound probe pose information may simply consist of an identifier of a particular ultrasound image 15 in the data stream received from the first location 100 such that the relevant ultrasound probe pose may be retrieved at the first location 100 by retrieving the metadata 16 corresponding to the identified particular ultrasound image 15. Alternatively, the target ultrasound probe pose information may contain the metadata 16 extracted from the received data stream that corresponds to the ultrasound image 15 in that the data stream as selected by the expert in the second location 150. In case of a re-sliced 2-D image slice 15', the target ultrasound probe pose information may comprise an identifier of an original 2-D image slice 15 in the received data stream together with ultrasound probe repositioning information generated by the processor 31, e.g. a transformation matrix or the like, which repositioning information typically contains information from which the pose of the ultrasound probe 11 as defined by the metadata 16 associated with the selected original 2-D image slice 15 may be transformed into the required pose for capturing re-sliced 2-D image slice 15' with the ultrasound probe 11. In this embodiment, the original pose of the ultrasound probe 11 may be transformed with the processor 21 of the ultrasound imaging guidance system 20 in the first location 100. Alternatively, the processor 31 may transform the relevant probe pose information and provide the transformed pose information of the ultrasound probe 11 to the ultrasound imaging guidance system 20 such that the processor 21 of the ultrasound imaging guidance system 20 does not need to perform the transformation but only needs to generate the virtual image 17 to be displayed on the display device 25.

The method 300 subsequently terminates in 313. Prior to termination, the method 300 may further include sharing a selected ultrasound image 15 or the re-sliced 2-D image slice 15' between the ultrasound imaging support system 30 in the second location 150 and the ultrasound imaging guidance system 20 in the first location 100 such that the expert in a second location 150 may interact with the medical practitioner in the first location 100, e.g. by highlighting regions of interest in the shared ultrasound image, for example using a crosshair, cursor, a colored shape such as a circle or box or the like, which may be used to assist the medical practitioner in the first location 100 to focus the generation of the ultrasound images with the ultrasound imaging system 10 on the appropriate anatomical feature (region of interest) of the patient 1.

It is noted for the avoidance of doubt that although the method 300 has been depicted as a series of sequential steps, it will be immediately apparent by the skilled person that at least some of the steps may alternatively be performed concurrently, i.e. in parallel.

Aspects of the method 200 and the method 300 may be provided in the form of a computer program product comprising a computer readable storage medium having computer readable program instructions embodied therewith for, when executed on the processor 21 of the ultrasound imaging guidance system 20 or on the processor 31 of the ultrasound imaging support system 30, cause these processors to implement the relevant steps of the method 200 and the method 300 respectively.

Aspects of the present invention may be embodied as an ultrasound imaging guidance system 20, an ultrasound imaging support system 30, a method or computer program product. Aspects of the present invention may take the form of a computer program product embodied in one or more computer-readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. Such a system, apparatus or device may be accessible over any suitable network connection; for instance, the system, apparatus or device may be accessible over a network for retrieval of the computer readable program code over the network. Such a network may for instance be the Internet, a mobile communications network or the like.

More specific examples (a non-exhaustive list) of the computer readable storage medium may include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present application, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out the methods of the present invention by execution on the processor 21 or 31 may be written in any combination of one or more programming languages, including an object oriented programming language such as Java, Smalltalk, C++ or the like and conventional procedural programming languages, such as the "C" programming language or similar programming languages. The program code may execute entirely on the processor 21 or 31 as a stand-alone software package, e.g. an app, or may be executed partly on the processor 21 or 31 and partly on a remote server. In the latter scenario, the remote server may be connected to the ultrasound imaging guidance system 20 or the ultrasound imaging support system 30 through any type of network, including a local area network (LAN) or a wide area network (WAN), or the connection may be made to an external computer, e.g. through the Internet using an Internet Service Provider.

Aspects of the present invention are described above with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions to be executed in whole or in part on the processor 21 of the ultrasound imaging guidance system 20 or on the processor 31 of the ultrasound imaging support system 30, such that the instructions create means for implementing the functions/acts specified in the flowchart and/or block diagram block or blocks. These computer program instructions may also be stored in a computer-readable medium that can direct the ultrasound imaging guidance system 20 or the ultrasound imaging support system 30 to function in a particular manner.

The computer program instructions may be loaded onto the processor 21 or the processor 31 to cause a series of operational steps to be performed on the processor 21 or the processor 31, to produce a computer-implemented process such that the instructions which execute on the processor 21 or the processor 31 provide processes for implementing the functions/acts specified in the flowchart and/or block diagram block or blocks. The computer program product may form part of an ultrasound imaging guidance system 20 or an ultrasound imaging support system 30, e.g. may be installed on the ultrasound imaging guidance system 20 or the ultrasound imaging support system 30.

## Claims

1. A system comprising a remote ultrasound imaging support system (30) and an ultrasound image guidance system (20); the ultrasound imaging guidance system (20) being for guiding an operator of an ultrasound imaging system (10) comprising an ultrasound probe (11), the ultrasound imaging guidance system comprising:
a transceiver (23) adapted to
receive a sequence of ultrasound images (15) generated by the operator with the ultrasound probe (11) from the ultrasound imaging system (10);
generate for each of the ultrasound images actual pose information (16) of the ultrasound probe (11) when capturing said ultrasound image; and
transmit a data stream to the remote ultrasound imaging support system (30), said data stream including the sequence of ultrasound images and an indication for each ultrasound image of the actual pose of the ultrasound probe; and
receive target ultrasound probe pose information from the remote ultrasound imaging support system (30), said target ultrasound probe pose information being generated by the remote ultrasound imaging support system from the transmitted data stream;
the ultrasound imaging guidance system (20) further comprising a processor (21) communicatively coupled to the transceiver and programmed to generate a virtual image (17) of the ultrasound probe in a pose corresponding to the target ultrasound probe pose information, and a display device (25) communicatively coupled to the processor and adapted to display the virtual image.

2. The system of claim 1, wherein the sequence of ultrasound images (15) comprises a sequence of 2-D slices for generating a 3-D ultrasound volume.

3. The system of claim 1 or 2, wherein the processor (21) is adapted to derive the indication of the actual pose (16) of the ultrasound probe (11) for each slice based on a patient body model.

4. The system of any of claims 1-3, further comprising a probe pose detector (27) adapted to generate the indication of the actual pose (16) of the ultrasound probe (11) when capturing an ultrasound image (15) in said sequence.

5. The system of claim 4, wherein the probe pose detector (27) comprises a camera adapted to capture an image of the actual pose of the ultrasound probe (11) when generating an ultrasound image (15) of said sequence.

6. The system of any of claims 1-5, wherein:
the transceiver (23) is further adapted to receive one of the ultrasound images (15) of said sequence from the remote location, said ultrasound image including a highlighted region; and
the display device (25) is further adapted to display the ultrasound image including the highlighted region.

7. An ultrasound imaging system (10) comprising an ultrasound probe (11) and the system of any of claims 1-6.

8. An ultrasound imaging support system (30) comprising:
a transceiver (33) adapted to receive a data stream including a sequence of ultrasound images (15) generated with an ultrasound probe (11) of an ultrasound imaging system (10) and an indication for each ultrasound image of the actual pose (16) of the ultrasound probe when capturing said ultrasound image;
a processor (31) communicatively coupled to the transceiver;
a display device (35) communicatively coupled to the processor; and
a user interface (37) communicatively coupled to the processor; wherein the processor is programmed to:
control the display device to display the sequence of ultrasound images;
receive a user input from the user interface indicative of an image selection (15, 15') from said sequence of ultrasound images; and
generate target ultrasound probe pose information from the received indications of the actual pose of the ultrasound probe and the received image selection,
wherein the transceiver is further adapted to transmit the target ultrasound probe pose to a remote ultrasound imaging guidance system (20) associated with the ultrasound imaging system.

9. The ultrasound imaging support system (30) of claim 8, wherein the user-specified image selection comprises a selected ultrasound image (15) from the sequence of ultrasound images or a 2-D image slice (15') for generating a 3-D ultrasound volume (18) defined by the sequence of ultrasound images.

10. The ultrasound imaging support system (30) of claim 8 or 9, wherein the processor (31) is further programmed to:
receive a further user input from the user interface (37) indicative of a selected area within a selected ultrasound image (15, 15') from said sequence of ultrasound images; and
generate a highlighted region in the selected ultrasound image corresponding to the selected area; and
wherein the transceiver (33) is further adapted to transmit the selected ultrasound image including the highlighted region to the remote ultrasound imaging guidance system (20).

11. A method (200) of guiding the operation of an ultrasound imaging system (10) comprising an ultrasound probe (11); the method comprising:
receiving (213) target ultrasound probe pose information derived from a data stream including a sequence of ultrasound images (15) generated with the ultrasound probe and an indication for each ultrasound image of the actual pose (16) of the ultrasound probe when capturing said ultrasound image from a remote ultrasound imaging support system (30);
generating (215) a virtual image (17) of the ultrasound probe in a pose corresponding to the target ultrasound probe pose information; and
displaying the virtual image.

12. The method (200) of claim 11, further comprising:
receiving the sequence of ultrasound images (15) from the ultrasound imaging system (10);
generating the actual pose information (16) of the ultrasound probe (11) for each of the ultrasound images; and
transmitting said data stream to a remote ultrasound imaging support system (30).

13. A computer program product comprising a computer readable storage medium having computer readable program instructions embodied therewith for, when executed on the processor (21) of the ultrasound imaging guidance system (20) of any of claims 1-5, cause the processor to implement the steps of the method (200) of claim 11 or 12.

14. A method (300) of generating guidance information for operating an ultrasound imaging system (10) comprising an ultrasound probe (11), the method comprising:
receiving (303) a data stream including a sequence of ultrasound images (15) generated with the ultrasound probe and an indication for each ultrasound image of the actual pose (16) of the ultrasound probe when capturing said ultrasound image;
displaying (305) the sequence of ultrasound images;
receiving (307) a user input indicative of an image selection from said sequence of ultrasound images, wherein the image selection comprises a selected ultrasound image (15) from the sequence of ultrasound images or an 2-D image slice (15') of a 3-D ultrasound volume defined by the sequence of ultrasound images;
generating (309) target ultrasound probe pose information from the received indications of the actual pose of the ultrasound probe and the received user input; and
transmitting (311) the target ultrasound probe pose information to a remote ultrasound imaging guidance system (20) associated with the ultrasound imaging system.

15. A computer program product comprising a computer readable storage medium having computer readable program instructions embodied therewith for, when executed on the processor (31) of the ultrasound imaging support system (30) of any of claims 8-10, cause the processor to implement the steps of the method (300) of claim 14.

## Patentansprüche

1. System das ein ferngesteuertes Ultraschallbildgebungs-Unterstützungssystem (30) und ein Ultraschallbild-Leitsystem (20) umfasst, wobei das Ultraschallbild-Leitsystem (20) dazu vorgesehen ist, einen Bediener eines
Ultraschallbildgebungs-Systems (10) anzuleiten, das eine Ultraschallsonde (11) umfasst, wobei das Ultraschallbildgebungs-System Folgendes umfasst:
einen Transceiver (23), der dazu ausgelegt ist:
eine Folge von Ultraschallbildern (15) zu empfangen, die vom Bediener mittels der Ultraschallsonde (11) vom Ultraschallbildgebungs-System (10) erzeugt wurden;
für jedes der Ultraschallbilder Daten zur tatsächlichen Haltung (16) der Ultraschallsonde (11) beim Aufnehmen des Ultraschallbildes zu erzeugen; und
einen Datenstrom an das entfernte Ultraschallbildgebungs-Unterstützungssystem (30) zu übertragen, wobei der Datenstrom die Folge von Ultraschallbildern und für jedes Ultraschallbild eine Angabe der tatsächlichen Haltung der Ultraschallsonde enthält; und
Daten zur Soll-Haltung der Ultraschallsonde vom entfernten Ultraschallbildgebungs-Unterstützungssystem (30) zu empfangen, wobei die erwähnten Daten zu Soll-Haltung der Ultraschallsonde durch das entfernte Ultraschallbildgebungs-Unterstützungssystem aus dem übertragenen Datenstrom erzeugt werden,
wobei das Ultraschallbild-Leitsystem (20) ferner Folgendes umfasst:
einen Prozessor (21), der kommunikationstechnisch mit dem Transceiver verbunden und so programmiert ist, dass er ein virtuelles Bild (17) der Ultraschallsonde in einer der Soll-Haltung der Ultraschallsonde entsprechenden Haltung erzeugt und
eine Anzeigevorrichtung (25), die kommunikationstechnisch mit dem Prozessor verbunden ist und dazu ausgelegt ist, das virtuelle Bild anzuzeigen.

2. System nach Anspruch 1, wobei die
Folge von Ultraschallbildern (15) eine Folge von 2D-Schichten zum Erzeugen eines 3D-Ultraschallvolumens umfasst.

3. System nach Anspruch 1 oder 2, wobei der
Prozessor (21) dazu ausgelegt ist, die Angabe der tatsächlichen Haltung (16) der Ultraschallsonde (11) für jede Schicht basierend auf einem Patienten-Körpermodell abzuleiten.

4. System nach einem der Ansprüche 1 bis 3, ferner umfassend einen Sondenhaltungs-Detektor (27), der dazu ausgelegt ist, die Angabe der tatsächlichen Haltung (16) der Ultraschallsonde (11) zu erzeugen, wenn ein Ultraschallbild (15) der erwähnten Folge aufgenommen wird.

5. System nach Anspruch 4, wobei der Sondenhaltungs-Detektor (27) eine Kamera umfasst, die dazu angepasst ist, ein Bild der tatsächlichen Haltung der Ultraschallsonde (11) aufzunehmen, wenn ein Ultraschallbild (15) der erwähnten Folge erzeugt wird.

6. System nach einem der Ansprüche 1 bis 5,
wobei:
der Transceiver (23) ferner dazu ausgelegt, eines der Ultraschallbilder (15) der Folge von dem entfernten Ort zu empfangen, wobei das Ultraschallbild eine hervorgehobene Region enthält; und
die Anzeigevorrichtung (25) ferner dazu ausgelegt ist, das Ultraschallbild einschließlich des hervorgehobenen Bereichs anzuzeigen.

7. Ultraschallbildgebungs-System (10) mit einer Ultraschallsonde (11) und dem System nach einem der Ansprüche 1 bis 6.

8. Ultraschallbildgebungs-Unterstützungssystem (30), das Folgendes umfasst:
einen Transceiver (33), der dazu ausgelegt ist, einen Datenstrom zu empfangen, der eine Folge von Ultraschallbildern (15) enthält, die mit einer Ultraschallsonde (11) eines Ultraschallbildgebungs-Systems (10) erzeugt wurden sowie für jedes Ultraschallbild eine Angabe der tatsächlichen Haltung (16) der Ultraschallsonde während der Aufnahme des Ultraschallbildes;
einen Prozessor (31), der kommunikationstechnisch mit dem Transceiver verbunden ist;
eine Anzeigevorrichtung (35), die kommunikationstechnisch mit dem Prozessor verbunden ist; und
eine Benutzerschnittstelle (37), die kommunikationstechnisch mit dem Prozessor verbunden ist,
wobei der Prozessor zu Folgendem programmiert ist:
Steuerung der Anzeigevorrichtung zur Anzeige der Folge von Ultraschallbildern;
Empfang einer Benutzereingabe von der Benutzerschnittstelle, die eine Bildauswahl (15, 15') aus der Folge von Ultraschallbildern angibt; und
Erzeugung von Daten zur Soll-Haltung der Ultraschallsonde aus den empfangenen Angaben der tatsächlichen Haltung der Ultraschallsonde und der empfangenen Bildauswahl,
wobei der Transceiver ferner dazu ausgelegt ist, die Soll-Haltung der Ultraschallsonde an ein entferntes Ultraschallbildgebungs-Leitsystem (20) zu senden, das mit dem Ultraschallbildgebungs-System verbunden ist.

9. Ultraschallbildgebungs-Unterstützungssystem (30) nach Anspruch 8, wobei die benutzerspezifische Bildauswahl ein ausgewähltes Ultraschallbild (15) aus der Folge von Ultraschallbildern oder eine 2D-Bildschichten (15') zum Erzeugen eines 3D-Ultraschallvolumen (18) umfasst, das durch die Folge von Ultraschallbildern definiert wird.

10. Ultraschallbildgebungs-Unterstützungssystem (30) nach Anspruch 8 oder 9, wobei der Prozessor (31) ferner zu Folgendem programmiert ist:
Empfang einer weiteren Benutzereingabe von der Benutzerschnittstelle (37), die einen ausgewählten Bereich innerhalb eines ausgewählten Ultraschallbildes (15, 15') aus der Folge von Ultraschallbildern angibt; und
Erzeugung eines hervorgehobenen Bereichs in dem ausgewählten Ultraschallbild, der dem ausgewählten Bereich entspricht; und
wobei der Transceiver (33) ferner dazu ausgelegt ist, das ausgewählte Ultraschallbild einschließlich des hervorgehobenen Bereichs zu dem entfernten Ultraschallbildgebungs-Leitsystem (20) zu übertragen.

11. Verfahren (200) zum Leiten des Betriebs eines Ultraschallbildgebungs-Systems (10), das eine Ultraschallsonde (11) umfasst, wobei das Verfahren Folgendes umfasst:
Empfang (213) von Daten zur Soll-Haltung der Ultraschallsonde, die aus einem Datenstrom mit einer Folge von Ultraschallbildern (15) abgeleitet sind, die mit der Ultraschallsonde erzeugt wurden, und für jedes Ultraschallbild einer Anzeige der tatsächlichen Haltung (16) der Ultraschallsonde, während das Ultraschallbild von einem entfernten Ultraschallbildgebungs-Unterstützungssystem (30) aufgenommen wird;
Erzeugung (215) eines virtuellen Bildes (17) der Ultraschallsonde in einer Haltung, die den Daten zur Soll-Haltung der Ultraschallsonde entspricht; und
Anzeige des virtuellen Bildes.

12. Verfahren (200) nach Anspruch 11, das ferner Folgendes umfasst:
Empfang der Folge von Ultraschallbildern (15) von dem Ultraschallbildgebungs-System (10);
Erzeugung der Daten zur tatsächlichen Haltung (16) der Ultraschallsonde (11) für jedes der Ultraschallbilder; und
Übertragung des Datenstroms an ein entferntes Ultraschallbildgebungs-Unterstützungssystem (30).

13. Computerprogramm-Produkt mit einem computerlesbaren Speichermedium, das computerlesbare Programmanweisungen enthält, die, wenn sie auf dem Prozessor (21) des Ultraschallbild-Leitsystems (20) nach einem der Ansprüche 1 bis 5 ausgeführt werden, den Prozessor veranlassen, Schritte des Verfahrens (200) nach Anspruch 11 oder 12 zu realisieren.

14. Verfahren (300) zum Erzeugen von Leit-Daten zum Betreiben eines Ultraschallbildgebungs-Systems (10) mit einer Ultraschallsonde (11), wobei das Verfahren Folgendes umfasst:
Empfang (303) eines Datenstroms, der eine Folge von Ultraschallbildern (15) enthält, die mit der Ultraschallsonde erzeugt werden sowie für jedes Ultraschallbild eine Angabe der tatsächlichen Haltung (16) der Ultraschallsonde, während das Ultraschallbild aufgenommen wird;
Anzeige (305) der Folge von Ultraschallbildern;
Empfang (307) einer Benutzereingabe, die eine Bildauswahl aus der Folge von Ultraschallbildern angibt, wobei die Bildauswahl ein ausgewähltes Ultraschallbild (15) aus der Folge von Ultraschallbildern oder eine 2D-Bildschicht (15') eines durch die Folge von Ultraschallbildern definierten 3D-Ultraschallvolumens enthält;
Erzeugung (309) von Daten zur Soll-Haltung der Ultraschallsonde aus den empfangenen Angaben der tatsächlichen Haltung der Ultraschallsonde und der empfangenen Benutzereingabe; und
Übertragung (311) der Daten zur Soll-Haltung der Ultraschallsonde an ein entferntes Ultraschallbildgebungs-Leitsystem (20), das mit dem Ultraschallbildgebungs-System verbunden ist.

15. Computerprogramm-Produkt mit einem computerlesbaren Speichermedium, das computerlesbare Programmanweisungen enthält, die, wenn sie auf dem Prozessor (31) des Ultraschallbild-Unterstützungssystems (30) nach einem der Ansprüche 8 bis 10 ausgeführt werden, den Prozessor veranlassen, Schritte des Verfahrens (300) nach Anspruch 14 zu realisieren.

## Revendications

1. Système comprenant un système de support de l'imagerie par ultrasons (30) distant et un système de guidage de l'imagerie par ultrasons (20) ; ledit système de guidage de l'imagerie par ultrasons (20) étant destiné au guidage d'un opérateur d'un système d'imagerie par ultrasons (10) comprenant une sonde ultrasonore (11), ledit système de guidage de l'imagerie par ultrasons comprenant :
un émetteur-récepteur (23) conçu pour
recevoir une séquence d'images ultrasonores (15) générée par l'opérateur à l'aide de la sonde ultrasonore (11) du système d'imagerie par ultrasons (10) ;
générer pour l'image ultrasonore respective des informations de pose (16) réelles de la sonde ultrasonore (11) lors de la capture de ladite image ultrasonore ; et
transmettre un flux de données au système de support de l'imagerie par ultrasons (30) distant,
ledit flux de données comprenant la séquence d'images ultrasonores et une indication pour l'image ultrasonore respective de la pose réelle de la sonde ultrasonore ; et
recevoir des informations de pose cible de la sonde ultrasonore du système de support de l'imagerie par ultrasons (30) distant, lesdites informations de pose cible de la sonde ultrasonore étant générées par le système de support de l'imagerie par ultrasons distant à partir du flux de données transmis ;
le système de guidage de l'imagerie par ultrasons (20) comprenant en outre
un processeur (21) couplé en communication à l'émetteur-récepteur et programmé pour générer une image (17) virtuelle de la sonde ultrasonore dans une pose correspondant aux informations de pose cible de la sonde ultrasonore, et
un dispositif d'affichage (25) couplé en communication au processeur et conçu pour afficher l'image virtuelle.

2. Système selon la revendication 1, dans lequel la séquence d'images ultrasonores (15) comprend une séquence de tranches 2D pour générer un volume ultrasonore 3D.

3. Système selon la revendication 1 ou 2, dans lequel le
processeur (21) est conçu pour dériver une indication de la pose (16) réelle de la sonde ultrasonore (11) pour la tranche respective en fonction d'un modèle corporel de patient.

4. Système selon l'une quelconque des revendications 1 à 3,
comprenant en outre un détecteur de pose de sonde (27) conçu pour générer une indication de la pose (16) réelle de la sonde ultrasonore (11) lors de la capture d'une image ultrasonore (15) dans ladite séquence.

5. Système selon la revendication 4, dans lequel le détecteur de pose de sonde (27) comprend une caméra conçue pour capturer une image de la pose réelle de la sonde ultrasonore (11) lors de la génération d'une image ultrasonore (15) de ladite séquence.

6. Système selon l'une quelconque des revendications 1 à 5,
dans lequel :
l'émetteur-récepteur (23) est en outre conçu pour recevoir l'une des images ultrasonores (15) de ladite séquence à partir d'un emplacement éloigné, ladite image ultrasonore comprenant une zone de surbrillance ; et
le dispositif d'affichage (25) est en outre conçu pour afficher l'image ultrasonore comprenant la zone de surbrillance.

7. Système d'imagerie par ultrasons (10) comprenant une sonde ultrasonore (11) et le système selon l'une quelconque des revendications 1 à 6.

8. Système de support de l'imagerie par ultrasons (30) distant comprenant :
un émetteur-récepteur (33) conçu pour recevoir un flux de données comprenant une séquence d'images ultrasonores (15) générée à l'aide d'une sonde ultrasonore (11) d'un système d'imagerie par ultrasons (10) et une indication pour l'image ultrasonore de la pose (16) réelle de la sonde ultrasonore lors de la capture de ladite image ultrasonore ;
un processeur (31) couplé en communication avec l'émetteur-récepteur ;
un dispositif d'affichage (35) couplé en communication avec le processeur ; et
une interface utilisateur (37) couplée en communication avec le processeur ; le processeur étant programmé :
pour commander le dispositif d'affichage pour afficher la séquence d'images ultrasonores ;
pour recevoir une entrée utilisateur de l'interface utilisateur indiquant une sélection d'image (15, 15') à partir de ladite séquence d'images ultrasonores ; et
pour générer des informations de pose cible de la sonde ultrasonore à partir des indications reçues de la pose réelle de la sonde ultrasonore et de la sélection d'images reçue,
l'émetteur-récepteur étant en outre conçu pour transmettre la pose cible de la sonde ultrasonore à un système de guidage de l'imagerie par ultrasons (20) distant associé au système d'imagerie par ultrasons.

9. Système de support de l'imagerie par ultrasons (30) distant selon la revendication 8, dans lequel la sélection d'image spécifiée par l'utilisateur comprend une image ultrasonore (15) sélectionnée à partir de la séquence d'images ultrasonores ou d'une tranche d'image (15') 2D pour générer un volume ultrasonore (18) 3D défini par la séquence d'images ultrasonores.

10. Système de support de l'imagerie par ultrasons (30) distant selon la revendication 8 ou 9, dans lequel le processeur (31) est en outre programmé :
pour recevoir une autre entrée utilisateur de l'interface utilisateur (37) indicative d'une zone sélectionnée dans une image ultrasonore (15, 15') sélectionnée à partir de ladite séquence d'images ultrasonores ; et
pour générer une zone de surbrillance dans l'image ultrasonore sélectionnée correspondant à la zone sélectionnée ; et
l'émetteur-récepteur (33) étant en outre conçu pour transmettre l'image ultrasonore sélectionnée comprenant la zone de surbrillance au système de guidage de l'imagerie par ultrasons (20).

11. Procédé (200) de guidage du fonctionnement d'un système d'imagerie par ultrasons (10) comprenant une sonde ultrasonore (11) ; ledit procédé comprenant :
la réception (213) des informations de pose cible de la sonde ultrasonore dérivées d'un flux de données comprenant une séquence d'images ultrasonores (15) générées à l'aide de la sonde ultrasonore et une indication pour l'image ultrasonore respective de la pose (16) réelle de la sonde ultrasonore lors de la capture de ladite image ultrasonore à partir d'un système de support de l'imagerie par ultrasons (30) distant ;
la génération (215) d'une image (17) virtuelle de la sonde ultrasonore dans une pose correspondant aux informations de pose cible de la sonde ultrasonore ; et
l'affichage de l'image virtuelle.

12. Procédé (200) selon la revendication 11, comprenant en outre :
la réception de la séquence d'images ultrasonores (15) du système d'imagerie par ultrasons (10) ;
la génération des informations de pose (16) réelle de la sonde ultrasonore (11) pour l'image ultrasonore respective ; et
la transmission dudit flux de données à un système de support de l'imagerie par ultrasons (30) distant.

13. Produit de programme informatique comprenant un support d'enregistrement lisible par ordinateur comportant des instructions de programme lisible par ordinateur incorporées pour amener, lorsque ledit programme est exécuté sur le processeur (21) du système de guidage de l'imagerie par ultrasons (20) selon l'une quelconque des revendications 1 à 5, le processeur à mettre en œuvre les étapes du procédé (200) selon la revendication 11 ou 12.

14. Procédé (300) de génération des informations de guidage destinées au fonctionnement d'un système d'imagerie par ultrasons (10) comprenant une sonde ultrasonore (11), ledit procédé comprenant :
la réception (303) d'un flux de données comprenant une séquence d'images ultrasonores (15) générée à l'aide de la sonde ultrasonore et une indication pour l'image ultrasonore respective de la pose (16) réelle de la sonde ultrasonore lors de la capture de ladite image ultrasonore ;
l'affichage (305) de la séquence d'images ultrasonores ;
la réception (307) d'une entrée utilisateur indicative d'une sélection d'image à partir de ladite séquence d'images ultrasonores, la sélection d'image comprenant une image ultrasonore (15) sélectionnée à partir de la séquence d'images ultrasonores ou une tranche (15') d'image 2D d'un volume ultrasonore 3D défini par la séquence d'images ultrasonores ;
la génération (309) des informations de pose cible de la sonde ultrasonore à partir des indications reçues de la pose réelle de la sonde ultrasonore et de l'entrée utilisateur reçue ; et
la transmission (311) des informations de pose cible de la sonde ultrasonore à un système de guidage de l'imagerie par ultrasons (20) distant associé au système d'imagerie par ultrasons.

15. Produit de programme informatique comprenant un support d'enregistrement lisible par ordinateur comportant des instructions de programme lisible par ordinateur incorporées pour amener, lorsque ledit programme est exécuté sur le processeur (31) du système de support de l'imagerie par ultrasons (30) distant selon l'une quelconque des revendications 8 à 10, le processeur à mettre en œuvre les étapes du procédé (300) selon la revendication 14.
